# EUROPEAN PATENT SPECIFICATION

(11) **EP 2 781 918 B1**
(45) Date of publication and mention of the grant of the patent: **28.06.2017**
(21) Application number: 12849967.0
(22) Date of filing: 04.01.2012
(51) Int. Cl.: G01N 33/543, G01N 33/532

(54) **MEANS FOR RAPIDLY DETECTING ADIPSIN FOR DIAGNOSING PREECLAMPSIA, TEST KIT AND PREPARATION METHOD THEREOF**
MITTEL ZUM SCHNELLEN NACHWEIS VON ADIPSIN ZUR DIAGNOSE VON PRÄEKLAMPSIE, TESTSATZ UND HERSTELLUNGSVERFAHREN DAFÜR
MOYENS POUR LA DÉTECTION RAPIDE D'ADIPSINE POUR LE DIAGNOSTIC DE LA TOXÉMIE PRÉÉCLAMPTIQUE, TROUSSE D'ESSAI ET SON PROCÉDÉ DE PRÉPARATION

(30) Priority: 17.11.2011 CN 201110365180
(43) Date of publication of application: 24.09.2014
(73) Proprietor: Origissay Biologics Technology Co. Ltd, Chengdu, Sichuan 610041 (CN)
(72) Inventor: HU, Huaizhong, Chengdu Sichuan 610041 (CN)
(74) Representative: Teschemacher, Andrea
(86) International application number: PCT/CN2012/070011
(87) International publication number: WO 2013/071703

(56) References cited:
- WO-A1-2009/097584
- WO-A2-2004/014220
- CN-A- 101 363 863
- CN-A- 101 592 666
- CN-A- 101 871 943
- CN-U- 202 351 243
- JP-A- 2004 041 208
- US-A- 5 622 871
- US-A1- 2007 172 963
- WANG, T ET AL.: "Elevatin of urinary adipsin in preeclampsia: correlation with urine protein concentration and the potential use for a rapid diagnostic test.", HYPERTENSION, [Online] vol. 64, no. 4, 23 June 2014 (2014-06-23), pages 846-851, XP009183720,
- MAVRI, A. ET AL.: 'Impact of Adipose Tissue on Plasma Plasminogen Activator Inhibitor-1 in Dieting Obese Women' ARTEROSCLEROSIS, THROMBOSIS, AND VASCULAR BIOLOGY vol. 19, no. 6, June 1999, pages 1582 - 1587, XP004920716
- NISHIZAWA, H. ET AL.: 'Microarray analysis of differentially expressed fetal genes in placental tissue derived from early and late onset severe pre-eclampsia' PLACENTA vol. 28, no. 5-6, June 2007, pages 487 - 497, XP022045824
- LYNCH, A.M. ET AL.: 'The interrelationship of complement-activation fragments and angiogenesis-related factors in early pregnancy and their association with pre-eclampsia' JOURNAL OF OBSTETRICS AND GYNAECOLOGY 14 January 2010, XP055069378

## Description

The present application claims the priority of China Patent Application No.201110365180.x, filed with the China Patent Office on November 17, 2011, titled "MEANS FOR RAPIDLY DETECTING ADIPSIN FOR DIAGNOSING PREECLAMPSIA, TEST KIT AND PREPARATION METHOD THEREOF".

### TECHNICAL FIELD OF THE INVENTION

The present invention belongs to the field of medical detection tool, particularly relates to a detection tool for detecting preeclampsia (abbreviated as PE) in pregnant women.

### BACKGROUND OF THE INVENTION

Preeclampsia (abbreviated as PE) is an obstetric complication occurring after 20 weeks of gestation, which is one of the main death causes of pregnant woman and prenatal infant. All over the world, the morbidity of preeclampsia in pregnant/puerperal women is 2∼8%, and the morbidity in pregnant/puerperal women in China may reach 10%. Severe preeclampsia patients may show clinical manifestations including hypertension, edema, severe proteinuria and the like, and are also easily complicated with adverse outcomes including pulmonary edema, renal failure, HELLP syndrome, etc. Furthermore, the risks of long-term suffering from cardiovascular diseases, kidney diseases and metabolic diseases are obviously increased. For the fetus, the less the gestational week is, the lower the survival rate is. The mortality for prematures before pregnancy week 32 is up to 50%. For newborns, the probability of severe complication including suffocation, intracranial hemorrhage, infection and the like is 5∼10%. The morbidity of long term metabolic diseases and cardiovascular diseases are also increased. All of these severely affect the health of offsprings, and bring heavy psychological and economic burden on families and society.

Prior art relating to the clinical diagnose requires to meet the following three conditions: (1) the pregnant/puerperal woman states that she has the clinical symptom of headache, dizziness and the like by herself; (2) the blood pressure of pregnant/puerperal woman detected by clinician meets the standard of hypertension, i.e. systolic pressure/diastolic pressure ≥140/90 mmHg; (3) the quantitative detection result of urinary albumin over 24 h is proteinuria ≥ 0.3g/day over 24 h. The quantitative detection of urinary albumin over 24 h needs to continuously collect the urine sample of a patient for 24 h for detection. This sample colleting method for such a process is complex and time-consuming.

Adipsin (also called factor D, or complement factor D) is a serine protease produced by adipocytes, which exists in circulating blood and is closely related to the fat metabolism in the body. The adipsin contents in the circulating blood of healthy pregnant women and the pregnant women suffering from preeclampsia are stable, and there is no significant difference between the two groups. However, since the pregnant women suffering from preeclampsia have vascular endothelium damage and the damage to vascular endothelial cells in kidney causes impaired penetration and recovery functions in kidney, this leads to the appearance of high concentration of adipsin in the urine sample of the pregnant women suffering from preeclampsia. Thus, adipsin can be used as an indicator for preeclampsia diagnoses and prognosis, based on this study basis. Currently, such detection tool and test kit which use adipsin content in urine sample as a detection indicator for preeclampsia have not been reported.

### SUMMARY OF THE INVENTION

The purpose of the present invention is to overcome the defects in the prior art and to provide a tool for rapidly detecting preeclampsia and test kit using adipsin in urine sample as the detection indicator, and a preparation method for the tool for rapidly detecting preeclampsia, in order to achieve noninvasive detection and to be able to rapidly obtain the detection results.

The inventor of the present patent application have found via experiments that adispin content is stable in the circulating blood of health pregnant women and the pregnant women suffering from preeclampsia, which is 2929.37±814.08 ng/mL in the blood of preeclampsia group, while 2359.76±667.63 ng/mL in healthy pregnant group, indicting there is no significant difference between the two groups. However, since the pregnant women suffering from preeclampsia have vascular endothelium damage, and the damage to vascular endothelial cells in kidney causes impaired penetration and recovery functions in kidney, this leads to appearance of high concentration of adipsin in the urine sample of the pregnant women suffering from preeclampsia. It is found in protein chip screening experiment that there is significant difference between the concentration of adipsin in the urine sample of the diagnosed preeclampsia puerperal women and the diagnosed non-preeclampsia puerperal women (the ages of the two groups matching the pregnancy week, no basic disease and no other complication of pregnancy). It is found in a quantitative study that the adipsin content is 349.04±557.10 ng/mL in the urine sample of preeclampsia group and is 8.69±6.34ng/mL in the urine sample of healthy pregnant group. There is significant difference between the preeclampsia group and healthy pregnant group, thus the adipsin content in urine sample can be used as the detection indicator to determine whether a pregnant woman suffers from preeclampsia.

The tool for rapidly detecting preeclampsia using adipsin as detection indicator according to the present invention comprises a base plate, a water-absorbing pad, a nitrocellulose membrane, a gold labeling pad and a sampling pad. The water-absorbing pad, nitrocellulose membrane, gold labeling pad and sampling pad are successively joined from top to bottom and fixed on the base plate, and the gold labeling pad is partly overlapped by the sampling pad; a detection line coated by rabbit anti-human adipsin polyclonal antibody, or goat anti-human adipsin polyclonal antibody, or mouse anti-human adipsin monoclonal antibody and a control line coated by goat anti-mouse IgG (immunoglobulin G, IgG) polyclonal antibody, are provided on the nitrocellulose membrane, the detection line is located under and spaced from the control line; the gold labeling pad is made of a water absorbing material, including glass fiber, polyester fiber membrane, non-woven fabrics or cellulose filter paper, and coated by a pad with gold labeled mouse anti-human adipsin monoclonal antibody conjugate.

The rapid test kit for preeclampsia using adipsin as detection indicator according to the present invention is composed of a housing and the aforementioned detection tool encased therein. The inner cavity of the housing matches the shape and size of the detection tool. The side wall of the housing is provided with a loading hole and an observation hole. The position of the loading hole is corresponding to the position of the sampling pad of the detection tool after being encased into the housing, and the position of the observation hole is corresponding to the position of the detection line and control line of the detection tool after being encased into the housing.

The housing can be monolithic structure and also can be separate structure. From the view of being convenient for installing the detection tool, a housing in separate structure is preferred in the present invention. The housing described in the present invention is formed by combining two box bodies equipped with a groove cavity, the connection manner between the two box bodies can be snap joint, buckle connection, screwed connection and the like, preferably snap joint and buckle connection.

In the detection tool mentioned above, the width of the detection line and the control line is preferably 0.5 mm∼1 mm; the distance between the detection line and the control line is preferably 4 mm∼12 mm. There is no strict requirement for the shape of the detection tool, and from the view of cost-saving and convenient preparing, it is preferably in the shape of a rectangular strip. The water-absorbing pad is a pad made of absorbent paper, and the sampling pad is a pad made of glass fiber, polyester fiber membrane, cellulose filter paper or non-woven fabrics which have the water-absorbing function.

The preparation method of the tool for rapidly detecting preeclampsia using adipsin as detection indicator according to the present invention is as follows:
(1) coating of detection line and control line
   ① using a phosphate buffer (pH=7.2∼7.4), or a carbonate buffer (pH=8∼10), or a borate buffer (pH=8.3∼9.3), or a tris(hydroxymethyl)aminomethane salt buffer (Tris salt, pH=7.2∼7.4) as solvent, and using rabbit anti-human adipsin polyclonal antibody, or goat anti-human adipsin polyclonal antibody, or mouse anti-human adipsin monoclonal antibody as solute, to formulate a detection line solution of rabbit anti-human adipsin polyclonal antibody, or goat anti-human adipsin polyclonal antibody, or mouse anti-human adipsin monoclonal antibody, with a concentration of 0.75 mg/mL∼5 mg/mL; using a phosphate buffer (pH=7.2∼7.4), or a carbonate buffer (pH=8∼10), or a borate buffer (pH=8.3∼9.3), or tris(hydroxymethylamino) methane salt buffer (Tris salt, pH=7.2∼7.4) as solvent, and using goat anti-mouse immunoglobulin G polyclonal antibody as solute, to formulate a control line solution of goat anti-mouse immunoglobulin G polyclonal antibody, with a concentration of no less than 1 mg/mL;
   ②attaching the nitrocellulose membrane on the base plate, setting parameters of a dispenser in terms of the required width of and distance between the control line and the detection line, coating the control line solution and the detection line solution on the nitrocellulose membrane by the dispenser, and then drying the coated nitrocellulose membrane-base plate combination at 37 °C for at least 8 h;
(2) preparation of gold labeling pad
   ①taking chlorauric acid and dissolving it in triple-distilled water to form an aqueous solution of chlorauric acid with a chlorauric acid concentration of 0.1∼0.2 g/100 mL, and heating the solution until boiling, adding an aqueous solution of trisodium citrate with a concentration of 0.9∼1.1 g/100 mL to the boiling aqueous solution of chlorauric acid under stirring (the volume ratio of the aqueous solution of trisodium citrate to the aqueous solution of chlorauric acid is 1.35∼1.5:50), stopping heating when the mixed liquid shows transparent wine red, after naturally cooling to room temperature, adjusting it to pH 6∼7.5 with K₂CO₃ solution, to obtain a colloidal gold solution;
   ②using a phosphate buffer (pH=7.2∼7.4), or tris(hydroxymethyl) aminomethane salt buffer as solvent, and using mouse anti-human adipsin monoclonal antibody as solute, to formulate the solution of mouse anti-human adipsin monoclonal antibody with a concentration of 0.1 mg/mL∼2 mg/mL, mixing the solution of mouse anti-human adipsin monoclonal antibody with the colloidal gold solution prepared in step ① (in this mixed liquid, the concentration of mouse anti-human adipsin monoclonal antibody is 10 µg/mL∼20 µg/mL), after evenly mixing, labeling the mixed liquid by a shaker for 20 min∼30 min, then adding a solution of bovine serum albumin and continuing labeling by shaker for 20 min∼30 min (the solution of bovine serum albumin is added in an amount that the concentration of bovine serum albumin in the mixed liquid reaches 0.1 g/100 mL), after completing the labeling, centrifuging at 8000 rpm∼12,000 rpm for 30 min∼40 min, to obtain the separated product, i.e. a gold labeled mouse anti-human adipsin monoclonal antibody conjugate, which is dissolved in a gold labeling complex solution to formulate a solution of the gold labeled mouse anti-human adipsin monoclonal antibody conjugate with a concentration of 0.2 mg/mL∼0.4 mg/mL (the gold labeling complex solution is formed by dissolving tris(hydroxymethyl)aminomethane, bovine serum albumin and sodium azide in double distilled water, in which the concentration of tris(hydroxymethyl)aminomethane is 0.01 mol/L∼0.02 mol/L, the concentration of bovine serum albumin is 0.1 g/100 mL∼1 g/100 mL and the concentration of sodium azide is 0.2 g/100 mL);
   ③spraying the solution of the gold labeled mouse anti-human adipsin monoclonal antibody conjugate on a water-absorbing material used for preparing gold labeling pad by a gold labeling sprayer, then drying the water-absorbing material sprayed with the solution of the gold labeled mouse anti-human adipsin monoclonal antibody conjugate at 37 °C for at least 8 h, to obtain the gold labeling pad;
(3) combination
   fixing the water-absorbing pad, the gold labeling pad and the sampling pad on the base plate in the coated nitrocellulose membrane-base plate combination, to form the tool for rapidly detecting; the fixed positions of the water-absorbing pad, the gold labeling pad and the sampling pad are to make the water-absorbing pad, the nitrocellulose membrane, the gold labeling pad and the sampling pad be successively joined from top to bottom, and the gold labeling pad is partly overlapped by the sampling pad.

In above method, for formulating the detection line solution and the control line solution, the concentration of phosphate buffer is 0.01 mol/L∼0.02 mol/L, the concentration of carbonate buffer is 0.05 mol/L, the concentration of borate buffer is 0.005 mol/L and the concentration of tris(hydroxymethyl)aminomethane salt buffer is 0.05 mol/L; for formulating the solution of mouse anti-human adipsin monoclonal antibody, the concentration of phosphate buffer is 0.01 mol/L∼0.02 mol/L and the concentration of tris(hydroxymethyl)aminomethane salt buffer is 0.05 mol/L.

In method mentioned above, the water-absorbing material for preparing gold labeling pad is one of glass fiber, polyester fiber membrane, non-woven fabrics and cellulose filter paper, the water-absorbing pad is made of water-absorbing paper, and the sampling pad is made of glass fiber, polyester fiber membrane, non-woven fabrics or cellulose filter paper; the spray amount of gold sprayer is preferably 2 µL/cm²∼4 µL/cm².

Using the detection tool and test kit according to the present invention, when one clear purple line is observed in the position of control line, demonstrating the detection tool according to the present invention is effective; in the case that the detection tool according to the present invention is effective, when one clear purple line is observed at the position of the detection line, such result is considered as positive(i.e. suffering from preeclampsia); if there is no purple line at the position of the detection line, then it is determined as negative(i.e. not suffer preeclampsia).

The detection tool and test kit according to the present invention have the following beneficial effects:
1. The present invention provides a novel detection tool employing a new detection indicator for detecting preeclampsia.
2. The test kit according to the present invention are used to carry out sampling detection for diagnosed preeclampsia group and diagnosed healthy pregnant women, and the results indicate that the sensitivity of the test kit is around 90% and the specificity of the test kit is around 80%.
3. The test kit described in the present invention is simply operated, which needs only 5∼10 min to detect one sample, and the detecting speed is substantially increased as compared to the existing detecting methods commonly used in clinic.
4. Since the detecting sample is the urine sample of pregnant women, it is a noninvasive detection.
5. The structure of the detection tool and test kit according to the present invention is simple, which are easy to be processed and manufactured with low production cost and are convenient for industrial production.

The detection tool and test kit according to the present invention are applicable to all of the pregnant women.

### DESCRIPTION OF THE DRAWINGS

Figure 1 is a structural schematic diagram of the tool for rapidly detecting preeclampsia using adipsin as the detection indicator according to the present invention;
Figure 2 is the left view of Figure 1;
Figure 3 is a structural schematic diagram of the test kit for rapidly detecting preeclampsia using adipsin as the detection indicator according to the present invention;
Figure 4 is the A-A sectional view of Figure 3;
Figure 5 is a structural schematic diagram of the test kit for rapidly detecting preeclampsia using adipsin as the detection indicator according to the present invention.

In the figures, 1-base plate, 2-water-absorbing pad, 3-control line, 4-detecing line, 5-nitrocellulose membrane, 6-gold labeling pad, 7-sampling pad, 8-housing (8-1: first box body, 8-2: second box body), 9-observation hole, 10-loading hole, 11-detection tool, 12-adipsin in the sample to be detected.

### DETAILED EMBODIMENTS

For further understanding the present invention, the prefer embodiments of the present invention is described below in connection with Examples, however, it should be understood that these descriptions are only for further illustrating the features and advantages of the present invention, rather than limiting the claims of the present invention.

The structure, preparation method and application method of the tool for rapidly detecting preeclampsia and test kit using adipsin as detection indicator according to the present invention are further illustrated in conjuction with the drawings.

The main materials and equipments relating to following Examples are as follows:
rabbit anti-human adipsin polyclonal antibody, purchased from RayBiotec corporation, U.S.;
goat anti-human adipsin polyclonal antibody, purchased from R&D corporation, U.S.;
mouse anti-human adipsin monoclonal antibody, purchased from R&D corporation, U.S.;
goat anti-mouse immunoglobulin G polyclonal antibody, purchased from BOSTER corporation, Wuhan;
carbonate buffer(CB), purchased from sigma corporation, U.S.;
phosphate buffer(PBS), purchased from sigma corporation, U.S.; tris(hydroxymethyl)aminomethane salt buffer(Tris salt buffer), purchased from sigma corporation, U.S.;
bovine serum albumin, purchased from sigma corporation, U.S.;
gold labeling sprayer, Model HGS510-1, Hangzhou Autokun Technology Co., Ltd; continuous dispenser, Model HGS101-2, HangzhouAutokun Technology Co., Ltd.

### Example 1

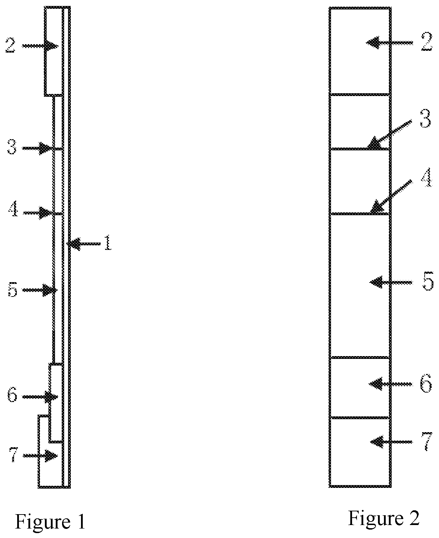

In this Example, the tool for rapidly detecting preeclampsia using adipsin as detection indicator was as shown in Figure 1 and Figure 2, which was in the shape of a rectangular strip and its configuration comprised a base plate 1, a water-absorbing pad 2, a nitrocellulose membrane 5, a gold labeling pad 6 and a sampling pad 7, wherein the water-absorbing pad, nitrocellulose membrane, gold labeling pad and sampling pad were successively joined from top to bottom and fixed on the base plate, and the gold labeling pad was partly overlapped by the sampling pad. Base plate 1 was made of medical plastic high whiteness PVC sheets, with pressure sensitive adhesive spread on it and inertia to bioactive molecular; water-absorbing paper with good water-absorbing performance was selected as water-absorbing pad 2, Model H5076; detection line 4 coated by rabbit anti-human adipsin polyclonal antibody and control line 3 coated by goat anti-mouse IgG polyclonal antibody were provided on the nitrocellulose membrane 5, the detection line 4 was located under the control line 3, the width of which were both 0.6 mm (or 0.8 mm or 0.9 mm) and the distance between them were 5 mm(or 4 mm or 6 mm or 8 mm or 12 mm); gold labeling pad 6 was made of glass fiber and coated by pad with gold labeled mouse anti-human adipsin monoclonal antibody conjugate; sampling pad was a pad which is made of glass fiber.

In this Example, the preparation method of the tool for rapidly detecting preeclampsia using adipsin as detection indicator was as follows:
(1) coating of detection line and control line
   ①A phosphate buffer with pH=7.2 and concentration of 0.01 mol/L was used as solvent, and rabbit anti-human adipsin polyclonal antibody was used as solute, to formulate a detection line solution of rabbit anti-human adipsin polyclonal antibody with a concentration of 1.5 mg/mL; the phosphate buffer with pH=7.2 and a concentration of 0.01 mol/L was used as solvent, and goat anti-mouse immunoglobulin G (abbreviated as goat anti-mouse IgG) polyclonal antibody was used as solute, to formulate a control line solution of goat anti-mouse IgG polyclonal antibody with a concentration of 1 mg/mL.
   ②The nitrocellulose membrane was attached on the sheet base plate material, the sheet base plate material attached with nitrocellulose membrane were placed on a dispenser, a control line/detection line distance standard card was put on one side of the sheet base plate material to determine the position of control line/detection line when coating (dispensing), parameters of a continuous dispenser were set in terms of the required width of and distance between the control line 3 and the detection line 4, and then the control line solution and the detection line solution were coated on the nitrocellulose membrane by operating the continuous dispenser.
   ③The coated (dispensed) nitrocellulose membrane-sheet base plate material combination was dried at 37 °C for 12 h with an electrothermal constant-temperature drying oven.
(2) Preparation of gold labeling pad
   ①Chlorauric acid was taken and dissolved in triple-distilled water to form an aqueous solution of chlorauric acid with a chlorauric acid concentration of 0.2 g/100 mL, and the solution was heated until boiling, a solution of trisodium citrate with a concentration of 1 g/100 mL was added to the aqueous solution of boiling chlorauric acid under stirring (the volume ratio of the aqueous solution of trisodium citrate to the aqueous solution of chlorauric acid is 1.4:50), stopped heating when the solution showed transparent wine red (heated for about 6 min), after naturally cooling to room temperature, adjusted it to pH 7.5 with K₂CO₃ solution, to obtain a colloidal gold solution (the mean particle diameter of the colloidal gold particle was 40 nm).
   ②A phosphate buffer with pH=7.2 and a concentration of 0.01 mol/L was used as solvent, and mouse anti-human adipsin monoclonal antibody was used as solute, to formulate a solution of mouse anti-human adipsin monoclonal antibody with a concentration of 1 mg/mL, 10 µL solution of mouse anti-human adipsin monoclonal antibody was mixed with 1mL the colloidal gold solution prepared in step (1), after evenly mixing, the mixture was labeled by shaker for 30 min with the centrifugal tube coated by foil; then 20 µL solution of bovine serum albumin with a concentration of 5 g/100 mL was added and mixed evenly, and continued labeling by shaker for 20 min. After labeling, it was centrifuged at 11,000 rpm for 30 min, the separated products obtained were gold labeled mouse anti-human adipsin monoclonal antibody conjugate, which was dissolved in 50 µL gold labeling complex solution to obtain a solution of the gold labeled mouse anti-human adipsin monoclonal antibody conjugate with a concentration of 0.2 mg/mL (stored at 4 °C for later use); the gold labeling complex solution was formed by tris(hydroxymethyl)aminomethane, bovine serum albumin and sodium azide dissolved in double distilled water, wherein the concentration of tris(hydroxymethyl)aminomethane is 0.02 mol/L, the concentration of bovine serum albumin is 1 g/100 mL and the concentration of sodium azide is 0.2 g/100 mL.
   ③The glass fibers for making gold labeling pad were cut into 7 cm×30 cm and put on the operation platform of a gold labeling sprayer, the spray amount was set as 4 µL/cm², the injection pump and pipeline of the gold labeling sprayer were filled with the solution of the gold labeled mouse anti-human adipsin monoclonal antibody conjugate, and the gold spaying operation was completed by operating the sprayer, the glass fibers sprayed with the solution of the gold labeled mouse anti-human adipsin monoclonal antibody conjugate were dried at 37 °C for 12 h with an electrothermal constant-temperature drying oven, to obtain the gold labeling pad.
(3) combination
   The sheet water-absorbing pad, sheet gold labeling pad and sheet sampling pad were attached on the sheet base plate material in the dispensed nitrocellulose membrane-sheet base plate material combination with pressure sensitive adhesive, then it was cut into 60 mm×4 mm rectangular strips which were the rapid detection tool described in the present Example; the attached position of sheet water-absorbing pad, sheet gold labeling pad and sheet sampling pad was to make the water-absorbing pad, nitrocellulose membrane, gold labeling pad and sampling pad be successively joined from top to bottom, and the gold labeling pad was partly overlapped by the sampling pad.

### Example 2

In this Example, the tool for rapidly detecting preeclampsia using adipsin as detection indicator was as shown in Figure 1 and Figure 2. The differences with Example 1 were that the detection line 4 on the nitrocellulose membrane 5 was coated by goat anti-human adipsin polyclonal antibody, the width of detection line 4 and control line 3 were both 0.5 mm(or 0.7 mm or 1 mm) and the distance between them were 5 mm(or 7 mm or 9 mm); gold labeling pad 6 was made of polyester fiber membrane and coated by gold labeled mouse anti-human adipsin monoclonal antibody conjugate; sampling pad was a pad which is made of polyester fiber membrane.

In this Example, the preparation method of the tool for rapidly detecting preeclampsia using adipsin as detection indicator was as follows:
(1) coating of detection line and control line
   ①A carbonate buffer with pH=9 and a concentration of 0.05 mol/L was used as solvent, and goat anti-human adipsin polyclonal antibody was used as solute, to formulate a detection line solution of goat anti-human adipsin polyclonal antibody with a concentration of 3.0 mg/mL; the carbonate buffer with pH=9 and a concentration of 0.05 mol/L was used as solvent, and goat anti-mouse immunoglobulin G (abbreviated as goat anti-mouse IgG) polyclonal antibody was used as solute, to formulate a control line solution of goat anti-mouse IgG polyclonal antibody with a concentration of 1 mg/mL.
   ②The coating operation was the same as that in Example 1.
(2) Preparation of gold labeling pad
   ①Chlorauric acid was taken and dissolved in triple-distilled water to form an aqueous solution of chlorauric acid with a chlorauric acid concentration of 0.1 g/100 mL, and the solution was heated until boiling, a solution of trisodium citrate with a concentration of 0.9 g/100 mL was added to the boiling aqueous solution of chlorauric acid under stirring (the volume ratio of the aqueous solution of trisodium citrate to the aqueous solution of chlorauric acid was 1.5:50), stopped heating when the solution showed transparent wine red (heated for about 6 min), after naturally cooling to room temperature, adjusted it to pH 7 with K₂CO₃ solution, to obtain a colloidal gold solution (the mean particle diameter of the colloidal gold particle is 40 nm).
   ②A tris(hydroxymethyl)aminomethane salt buffer with pH=7.4 and a concentration of 0.05 mol/L was used as solvent, and mouse anti-human adipsin monoclonal antibody was used as solute, to formulate a solution of mouse anti-human adipsin monoclonal antibody with a concentration of 0.6 mg/mL, the solution of mouse anti-human adipsin monoclonal antibody was mixed with the colloidal gold solution prepared in step (1), in the mixed liquid, the concentration of the mouse anti-human adipsin monoclonal antibody was 15 µg/mL, after evenly mixing, it was labeled by shaker for 20 min with the centrifugal tube coated by foil; then a solution of bovine serum albumin with a concentration of 5 g/100 mL was added and evenly mixed, and continued labeling by shaker for 30 min (the solution of bovine serum albumin is added in an amount that the concentration of bovine serum albumin reached 0.1 g/100 mL in the mixed liquid). After labeling, it was centrifuged at 10,000 rpm for 40 min, the separated products obtained were gold labeled mouse anti-human adipsin monoclonal antibody conjugate, which was dissolved in a gold labeling complex solution to formulate a solution of the gold labeled mouse anti-human adipsin monoclonal antibody conjugate with the a concentration of 0.3 mg/mL (stored at 4 °C for later use); the gold labeling complex solution is formed by tris(hydroxymethyl)aminomethane, bovine serum albumin and sodium azide dissolved in double distilled water, wherein the concentration of tris(hydroxymethyl)aminomethane was 0.01 mol/L, the concentration of bovine serum albumin was 0.5 g/100 mL and the concentration of sodium azide was 0.2 g/100 mL.
   ③The glass fibers for making gold labeling pad were cut into 7 cm×30 cm and put on the operation platform of a gold labeling sprayer, the spray amount was set as 3 µL/cm², the injection pump and pipeline of the gold labeling sprayer was filled with the solution of the gold labeled mouse anti-human adipsin monoclonal antibody conjugate, and the gold spaying operation was completed by operating the sprayer, the glass fibers sprayed with the solution of the gold labeled mouse anti-human adipsin monoclonal antibody conjugate were dried at 37 °C for 10 h with an electrothermal constant-temperature drying oven, to obtain the gold labeling pad.
(3) combination
   The combination manner was the same as that in Example 1.

### Example 3

In this Example, the tool for rapidly detecting preeclampsia using adipsin as detection indicator was as shown in Figure 1 and Figure 2. The differences with Example 1 were that the detection line 4 on the nitrocellulose membrane 5 was coated by mouse anti-human adipsin monoclonal antibody, the width of detection line 4 and control line 3 were both 0.5 mm or 0.8 mm and the distance between them were 6 mm or 10 mm; gold labeling pad 6 was made of non-woven fabrics and coated by gold labeled mouse anti-human adipsin monoclonal antibody conjugate; sampling pad was a pad which is made of non-woven fabrics.

In this Example, the preparation method of the tool for rapidly detecting preeclampsia using adipsin as detection indicator was as follows:
(1) coating of detection line and control line
   ①A tris(hydroxymethyl)aminomethane salt buffer with pH=7.4 and a concentration of 0.05 mol/L was used as solvent, and mouse anti-human adipsin monoclonal antibody was used as solute, to formulate a detection line solution of mouse anti-human adipsin polyclonal antibody with a concentration of 4.5 mg/mL; the tris(hydroxymethyl)aminomethane salt buffer with pH=7.4 and a concentration of 0.05 mol/L was used as solvent, and goat anti-mouse immunoglobulin G (abbreviated as goat anti-mouse IgG) polyclonal antibody was used as solute, to formulate a control line solution of goat anti-mouse IgG polyclonal antibody with a concentration of 1 mg/mL.
   ②The coating operation was the same as that in Example 1.
(2) Preparation of gold labeling pad
   ①Chlorauric acid was taken and dissolved in triple-distilled water to form an aqueous solution of chlorauric acid with a chlorauric acid concentration of 0.15 g/100 mL, and the solution was heated until boiling, a solution of trisodium citrate with a concentration of 1.1 g/100 mL was added to the boiling aqueous solution of chlorauric acid under stirring (the volume ratio of the aqueous solution of trisodium citrate to the aqueous solution of chlorauric acid is 1.35:50), stopped heating when the solution showed transparent wine red (heated for about 6 min), after naturally cooling to room temperature, adjusted it to pH 6.5 with K₂CO₃ solution, to obtain a colloidal gold solution (the mean particle diameter of the colloidal gold particle was 40 nm).
   ②A tris(hydroxymethyl)aminomethane salt buffer with pH=7.2 and a concentration of 0.05 mol/L was used as solvent, and mouse anti-human adipsin monoclonal antibody was used as solute, to formulate a solution of mouse anti-human adipsin monoclonal antibody with a concentration of 1.5 mg/mL, the solution of mouse anti-human adipsin monoclonal antibody was mixed with the colloidal gold solution prepared in step (1), in the mixed liquid, the concentration of mouse anti-human adipsin monoclonal antibody was 20 µg/mL, after evenly mixing, it was labeled by shaker for 25 min with the centrifugal tube coated by foil; then a solution of bovine serum albumin with a concentration of 5 g/100 mL was added and evenly mixed, and continued labeling by shaker for 25 min (the solution of bovine serum albumin was added in an amount that the concentration of bovine serum albumin reached 0.1 g/100 mL in the mixed liquid). After labeling, it was centrifuged at 8,000 rpm for 40 min, the separated products obtained were gold labeled mouse anti-human adipsin monoclonal antibody conjugate, which was dissolved in a gold labeling complex solution to formulate a solution of the gold labeled mouse anti-human adipsin monoclonal antibody conjugate with a concentration of 0.4 mg/mL (stored at 4 °C for later use); the gold labeling complex solution was formed by tris(hydroxymethyl)aminomethane, bovine serum albumin and sodium azide dissolved in double distilled water, wherein the concentration of tris(hydroxymethyl)aminomethane was 0.015 mol/L, the concentration of bovine serum albumin was 0.3 g/100 mL and the concentration of sodium azide was 0.2 g/100 mL.
   ③The glass fibers for making gold labeling pad were cut into 7 cm×30 cm and put on the operation platform of a gold labeling sprayer, the spray amount was set as 2 µL/cm², the injection pump and pipeline of the gold labeling sprayer was filled with the solution of the gold labeled mouse anti-human adipsin monoclonal antibody conjugate, and the gold spaying operation was completed by operating the sprayer, the glass fibers sprayed with the solution of the gold labeled mouse anti-human adipsin monoclonal antibody conjugate were dried at 37 °C for 12 h with an electrothermal constant-temperature drying oven, to obtain the gold labeling pad.
(3) combination
   The combination manner was the same as that in Example 1.

### Example 4

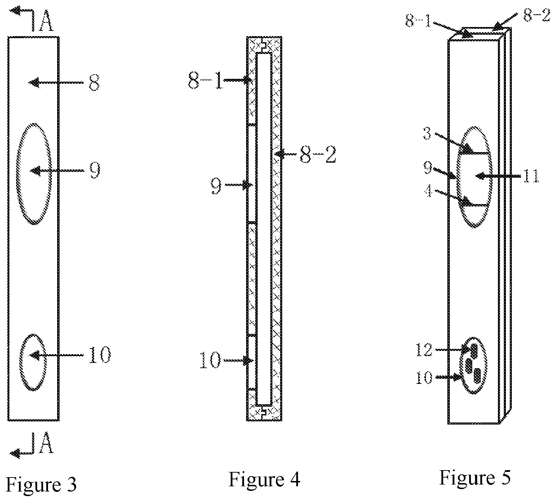

In this Example, the shape and configuration of the test kit for rapid detecting preeclampsia using adipsin as detection indicator was as shown in Figure 5, which was composed of housing 8 and the detection tool 11 encased therein. The detection tool 11 was the detection tool with the shape and configuration described in Example 1 or Example 2 or Example 3. The housing 8 was made of medical plastic and combined with the first box body 8-1 and the second box body 8-2 both equipped with a groove cavity, the connection manner between the two box bodies was snap joint, as shown in Figure 3 and Figure 4. The shape of the housing combined by the two box bodies was rectangular, and its inner cavity matched the shape and size of the detection tool. One side wall of the housing was provided with a loading hole 10 and an observation hole 9. The position of the loading hole 10 was corresponding to the position of the sampling pad 7 of the detection tool after being encased into the housing, and the position of the observation hole 9 was corresponding to the position of the detection line 4 and the control line 3 of the detection tool after being encased into the housing. The prepared test kit was sealed with the aluminum foil bag with drying agent in it for later use.

The application method and detection principle of the test kit were as follows:
The sample to be detected (urine sample from gestational woman) was added into sampling pad 7 of the detection tool through loading hole 10 in the housing 8. Due to chromatographic principle, the sample to be detected moved up along with the strip detection tool from sampling pad 7. When moving to gold labeling pad 6, the gold labeled mouse anti-human adipsin monoclonal antibody conjugate was dissolved and the sample continued to move up along with the gold labeled mouse anti-human adipsin monoclonal antibody. When moving to the detection line 4 on nitrocellulose membrane, if there were Adispin antigen existing in the sample to be detected, the rabbit anti-human adipsin polyclonal antibody, or goat anti-human adipsin polyclonal antibody, or mouse anti-human adipsin monoclonal antibody and gold labeled mouse anti-human adipsin monoclonal antibody formed one purple sandwich immune complex (with adipsin antigen in the sample at the position of detection line. One clear purple line could be observed at the position of detection line, and such result was considered as positive; the higher the adipsin antigen content was in the sample, the deeper the color of the color line was. If there were no adipsin antigen existing in the sample, gold labeled mouse anti-human adipsin monoclonal antibody would not form complex with the rabbit anti-human adipsin polyclonal antibody, or goat anti-human adipsin polyclonal antibody, or mouse anti-human adipsin monoclonal antibody of detection line. There was no purple line appearing at the position of detection line, and such result was determined as negative. When the sample to be detected and the gold labeled mouse anti-human adipsin monoclonal antibody continued to move up to the control line 3, the goat anti-mouse IgG polyclonal antibody on the control line would conjugate with gold labeled mouse anti-human adipsin monoclonal antibody to form one purple line on the position of the control line, which demonstrated that the detection tool was effective. The detection results could be obtained by observation though the observation hole 9 in the housing.

The tool for rapidly detecting preeclampsia and test kit, and the preparation method of the detection tool proposed in the present invention have been described through Examples. It is apparent that relative skilled staff could make alterations or proper modifications and combinations to the products and preparation method as described herein without departing from the contents, spirit and scope of the present invention to implement the technology of the present invention. In particular, all of the similar replacements and alternations are apparent to those skilled in the art, which are regarded as being included in the spirit, scope and contents of the present invention.

## Claims

1. Use of a tool for rapidly detecting preeclampsia in a urine sample of a pregnant women using adipsin as detection indicator, **characterized in that**
the tool comprises a base plate (1), a water-absorbing pad (2), a nitrocellulose membrane (5), a gold labeling pad (6) and a sampling pad (7), the water-absorbing pad, nitrocellulose membrane, gold labeling pad and sampling pad are successively joined from top to bottom and fixed on the base plate, and the gold labeling pad is partly overlapped by the sampling pad;
the nitrocellulose membrane (5) is provided with a detection line (4) coated by rabbit anti-human adipsin polyclonal antibody, or goat anti-human adipsin polyclonal antibody, or mouse anti-human adipsin monoclonal antibody, and a control line (3) coated by goat anti-mouse immunoglobulin G polyclonal antibody, the detection line is located under and spaced from the control line;
the gold labeling pad (6) is made of water absorbing material, and coated by a pad with gold labeled mouse anti-human adipsin monoclonal antibody conjugate.

2. Use of the tool for rapidly detecting preeclampsia using adipsin as detection indicator according to claim 1, **characterized in that** the width of the detection line (4) and the control line (3) is 0.5 mm∼1 mm.

3. Use of the tool for rapidly detecting preeclampsia using adipsin as detection indicator according to claim 1 or 2, **characterized in that** the distance between the detection line (4) and the control line (3) is 4 mm∼12 mm.

4. Use of the tool for rapidly detecting preeclampsia using adipsin as detection indicator according to claim 1 or 2, **characterized in that** it is in the shape of a rectangular strip.

5. Use of a test kit for rapidly detecting preeclampsia in a urine sample of a pregnant women using adipsin as detection indicator, **characterized in that**
the test kit is composed of a housing (8) and a detection tool encased therein;
the detection tool comprises a base plate (1), a water-absorbing pad (2), a nitrocellulose membrane (5), a gold labeling pad (6) and a sampling pad (7), the water-absorbing pad, the nitrocellulose membrane, the gold labeling pad and the sampling pad are successively joined from top to bottom and fixed on the base plate, and the gold labeling pad is partly overlapped by the sampling pad, the nitrocellulose membrane (5) is provided with a detection line (4) coated by rabbit anti-human adipsin polyclonal antibody, or goat anti-human adipsin polyclonal antibody, or mouse anti-human adipsin monoclonal antibody and a control line (3) coated by goat anti-mouse immunoglobulin G polyclonal antibody, the detection line is located under and spaced from the control line, the gold labeling pad (6) is glass fibers coated by gold labeled mouse anti-human adipsin monoclonal antibody conjugate;
the inner cavity of the housing (8) matches the shape and size of the detection tool, the side wall of the housing (8) is provided with a loading hole (10) and an observation hole (9), the position of the loading hole (10) is corresponding to the position of the sampling pad (7) of the detection tool after being encased into the housing, and the position of the observation hole (9) is corresponding to the position of the detection line (4) and the control line (3) of the detection tool after being encased into the housing.

6. Use of the test kit for rapidly detecting preeclampsia using adipsin as detection indicator according to claim 5, **characterized in that** the housing (8) is formed by combining two box bodies (8-1, 8-2) equipped with a groove cavity.

7. Use of the test kit for rapidly detecting preeclampsia using adipsin as detection indicator according to claim 6, **characterized in that** the connection manner between the two box bodies (8-1, 8-2) equipped with a groove cavity is snap joint or buckle connection.

## Patentansprüche

1. Verwendung eines Werkzeugs zum schnellen Nachweis von Präeklampsie in einer Urinprobe einer schwangeren Frau unter Verwendung von Adipsin als Nachweisindikator, **dadurch gekennzeichnet, dass**
das Werkzeug eine Grundplatte (1), ein wasserabsorbierendes Pad (2), eine Nitrozellulosemembran (5), ein Goldmarkierungspad (6) und ein Probeentnahmepad aufweist (7), wobei das wasserabsorbierende Pad, die Nitrozellulosemembran, das Goldmarkierungspad und das Probeentnahmepad nacheinander von oben nach unten verbunden und auf der Grundplatte fixiert sind, und das Goldmarkierungspad teilweise von dem Probeentnahmepad überlappt wird;
die Nitrozellulosemembran (5) mit einer Nachweislinie (4), welche mit Kaninchen-Anti-Human-Adipsin-polyklonalem Antikörper oder Ziegen-Anti-Human-Adipsin-polyklonalem Antikörper oder Maus-Anti-Human-Adipsin-monoklonalem Antikörper beschichtet ist, und mit einer Kontrolllinie (3) versehen ist, welche mit Ziegen-Anti-Maus-Immunglobulin-G-polyklonalem Antikörper beschichtet ist, wobei sich die Nachweislinie unter der Kontrolllinie befindet und von dieser beabstandet ist;
das Goldmarkierungspad (6) aus wasserabsorbierendem Material hergestellt ist und mit einem Pad mit goldmarkiertem Maus-Anti-Human-Adipsin-monoklonalem Antikörper-Konjugat beschichtet ist.

2. Verwendung des Werkzeugs zum schnellen Nachweis von Präeklampsie unter Verwendung von Adipsin als Nachweisindikator nach Anspruch 1, **dadurch gekennzeichnet, dass** die Breite der Nachweislinie (4) und der Kontrolllinie (3) 0,5 mm ∼ 1 mm beträgt.

3. Verwendung des Werkzeugs zum schnellen Nachweis von Präeklampsie unter Verwendung von Adipsin als Nachweisindikator nach Anspruch 1 oder 2, **dadurch gekennzeichnet, dass** der Abstand zwischen der Nachweislinie (4) und der Kontrolllinie (3) 4 mm ∼ 12 mm beträgt.

4. Verwendung des Werkzeugs zum schnellen Nachweis von Präeklampsie unter Verwendung von Adipsin als Nachweisindikator nach Anspruch 1 oder 2, **dadurch gekennzeichnet, dass** es in Form eines rechteckigen Streifens vorliegt.

5. Verwendung eines Testkits zum schnellen Nachweis von Präeklampsie in einer Urinprobe einer schwangeren Frau unter Verwendung von Adipsin als Nachweisindikator, **dadurch gekennzeichnet, dass**
das Testkit aus einem Gehäuse (8) und einem darin eingekapselten Nachweiswerkzeug besteht;
das Nachweiswerkzeug eine Grundplatte (1), ein wasserabsorbierendes Pad (2), eine Nitrozellulosemembran (5), ein Goldmarkierungspad (6) und ein Probeentnahmepad aufweist (7), wobei das wasserabsorbierende Pad, die Nitrozellulosemembran, das Goldmarkierungspad und das Probeentnahmepad nacheinander von oben nach unten verbunden und auf der Grundplatte fixiert sind, und das Goldmarkierungspad teilweise von dem Probeentnahmepad überlappt wird, wobei die Nitrozellulosemembran (5) mit einer Nachweislinie (4), welche mit Kaninchen-Anti-Human-Adipsin-polyklonalem Antikörper oder Ziegen-Anti-Human-Adipsin-polyklonalem Antikörper oder Maus-Anti-Human-Adipsin-monoklonalem Antikörper beschichtet ist, und mit einer Kontrolllinie (3) versehen ist, welche mit Ziegen-Anti-Maus-Immunglobulin-G-polyklonalem Antikörper beschichtet ist, wobei sich die Nachweislinie unter der Kontrolllinie befindet und von dieser beabstandet ist, wobei das Goldmarkierungspad (6) aus Glasfasern besteht, die mit goldmarkiertem Maus-Anti-Human-Adipsin- monoklonalem Antikörper-Konjugat beschichtet sind;
der innere Hohlraum des Gehäuses (8) der Form und Größe des Nachweiswerkzeugs entspricht, wobei die Seitenwand des Gehäuses (8) mit einem Beladeloch (10) und einem Beobachtungsloch (9) versehen ist, wobei die Position des Beladelochs (10) der Position des Probeentnahmepads (7) des Nachweiswerkzeugs nach dessen Einkapseln in dem Gehäuse entspricht, und wobei die Position des Beobachtungslochs (9) der Position der Nachweislinie (4) und der Kontrolllinie (3) des Nachweiswerkzeugs nach dessen Einkapseln in dem Gehäuse entspricht.

6. Verwendung des Testkits zum schnellen Nachweis von Präeklampsie unter Verwendung von Adipsin als Nachweisindikator nach Anspruch 5, **dadurch gekennzeichnet, dass** das Gehäuse (8) durch Kombination zweier mit einem Rillenhohlraum versehener Kastenkörper (8-1, 8-2) ausgebildet ist.

7. Verwendung des Testkits zum schnellen Nachweis von Präeklampsie unter Verwendung von Adipsin als Nachweisindikator nach Anspruch 6, **dadurch gekennzeichnet, dass** die Verbindungsart zwischen den beiden mit einem Rillenhohlraum versehenen Kastenkörpern (8-1, 8-2) ein Schnappverschluss oder eine Schnallenverbindung ist.

## Revendications

1. Utilisation d'un outil pour détecter rapidement une pré-éclampsie dans un échantillon d'urine d'une femme enceinte en utilisant de l'adipsine comme indicateur de détection, **caractérisée en ce que**
l'outil comprend une plaque de base (1), un tampon absorbant l'eau (2), une membrane de nitrocellulose (5), un tampon de marquage à l'or (6) et un tampon d'échantillonnage (7), le tampon absorbant l'eau, la membrane de nitrocellulose, le tampon de marquage à l'or et le tampon d'échantillonnage sont successivement reliés de haut en bas et fixés sur la plaque de base, et le tampon de marquage à l'or est partiellement recouvert par le tampon d'échantillonnage ;
la membrane de nitrocellulose (5) est munie d'une ligne de détection (4) revêtue d'un anticorps polyclonal anti-adipsine humaine de lapin ou d'un anticorps polyclonal anti-adipsine humaine de chèvre ou d'un anticorps monoclonal anti-adipsine humaine de souris, et d'une ligne témoin (3) revêtue d'un anticorps polyclonal de chèvre anti-immunoglobuline G de souris, la ligne de détection est située sous la ligne témoin et espacée de celle-ci ;
le tampon de marquage à l'or (6) est réalisé en un matériau absorbant l'eau, et revêtu d'un tampon avec un conjugué d'anticorps monoclonal anti-adipsine humaine de souris marqué à l'or.

2. Utilisation de l'outil pour détecter rapidement une pré-éclampsie en utilisant de l'adipsine comme indicateur de détection selon la revendication 1, **caractérisée en ce que** la largeur de la ligne de détection (4) et de la ligne témoin (3) est de 0,5 mm ∼ 1 mm.

3. Utilisation de l'outil pour détecter rapidement une pré-éclampsie en utilisant de l'adipsine comme indicateur de détection selon la revendication 1 ou 2, **caractérisée en ce que** la distance entre la ligne de détection (4) et la ligne témoin (3) est de 4 mm ∼ 12 mm.

4. Utilisation de l'outil pour détecter rapidement une pré-éclampsie en utilisant de l'adipsine comme indicateur de détection selon la revendication 1 ou 2, **caractérisée en ce qu'**il présente la forme d'une bande rectangulaire.

5. Utilisation d'un kit de test pour détecter rapidement une pré-éclampsie dans un échantillon d'urine d'une femme enceinte en utilisant de l'adipsine comme indicateur de détection, **caractérisée en ce que**
le kit de test est composé d'un boîtier (8) et d'un outil de détection enfermé dedans ;
l'outil de détection comprend une plaque de base (1), un tampon absorbant l'eau (2), une membrane de nitrocellulose (5), un tampon de marquage à l'or (6) et un tampon d'échantillonnage (7), le tampon absorbant l'eau, la membrane de nitrocellulose, le tampon de marquage à l'or et le tampon d'échantillonnage sont successivement reliés de haut en bas et fixés sur la plaque de base, et le tampon de marquage à l'or est partiellement recouvert par le tampon d'échantillonnage, la membrane de nitrocellulose (5) est munie d'une ligne de détection (4) revêtue d'un anticorps polyclonal anti-adipsine humaine de lapin ou d'un anticorps polyclonal anti-adipsine humaine de chèvre ou d'un anticorps monoclonal anti-adipsine humaine de souris, et d'une ligne témoin (3) revêtue d'un anticorps polyclonal de chèvre anti-immunoglobuline G de souris, la ligne de détection est située sous la ligne témoin et espacée de celle-ci, le tampon de marquage à l'or (6) est réalisé en des fibres de verre revêtues d'un conjugué d'anticorps monoclonal anti-adipsine humaine de souris marqué à l'or ;
la cavité interne du boîtier (8) correspond à la forme et à la taille de l'outil de détection, la paroi latérale du boîtier (8) est munie d'un trou de chargement (10) et d'un trou d'observation (9), la position du trou de chargement (10) correspond à la position du tampon d'échantillonnage (7) de l'outil de détection après avoir été enfermé dans le boîtier, et la position du trou d'observation (9) correspond à la position de la ligne de détection (4) et à la ligne témoin (3) de l'outil de détection après avoir été enfermé dans le boîtier.

6. Utilisation du kit de test pour détecter rapidement une pré-éclampsie en utilisant de l'adipsine comme indicateur de détection selon la revendication 5, **caractérisée en ce que** le boîtier (8) est formé en combinant deux corps de boîte (8-1, 8-2) équipés d'une cavité de rainure.

7. Utilisation du kit de test pour détecter rapidement une pré-éclampsie en utilisant de l'adipsine comme indicateur de détection selon la revendication 6, **caractérisée en ce que** le type de connexion entre les deux corps de boîte (8-1, 8-2) équipés d'une cavité de rainure est un joint à encliquetage ou une connexion à boucle.
